# EUROPEAN PATENT SPECIFICATION

(11) **EP 0 672 167 B1**
(45) Date of publication and mention of the grant of the patent: **10.12.1997**
(21) Application number: 93917910.7
(22) Date of filing: 23.07.1993
(51) Int. Cl.: C12Q 1/00, G01N 27/30, C12M 1/40

(54) **ANALYTICAL METHOD AND DEVICE FOR DETERMINATION OF HYDROGEN PEROXIDE**
ANALYTISCHES VERFAHREN UND VORRICHTUNG ZUR BESTIMMUNG VON HYDROGEN PEROXID
PROCEDE ET DISPOSITIF D'ANALYSE POUR LE PEROXIDE D'HYDROGENE

(30) Priority: 28.07.1992 GB 9216056
(43) Date of publication of application: 20.09.1995
(73) Proprietor: THE VICTORIA UNIVERSITY OF MANCHESTER, Manchester M13 9PL (GB)
(72) Inventor: VADGAMA, Pankaj Madganlal, Worsley, Manchester M28 4QA (GB); CHRISTIE, Ian McIntyre, Stockport, Cheshire SK12 7DG (GB); LLOYD, Sheridan James, Stanley, County Durham DH9 0XB (GB)
(74) Representative: Tunnicliffe, Peter Barry
(86) International application number: GB9301568
(87) International publication number: WO9402629

(56) References cited:
- EP-A- 0 177 743
- EP-A- 0 186 210
- EP-A- 0 239 969

## Description

This invention relates to an improved analytical method and apparatus, more particularly those useful for electro-analytical work and especially in amperometric measurements of hydrogen peroxide, and to analytical methods using them.

It is known to use electrodes in analytical procedures to detect and measure a variety of chemical species present in a liquid medium. The procedures may involve the measurement of various properties of the medium so that the presence of particular chemical species or groups of such species may be detected or measured. It is desirable to make these measurements as accurate, quantitative and selective as possible, so that the result of the measurements can be interpreted and used with maximum reliability.

Examples of electrode devices designed to serve special purposes include those described in:-
(a) European Patent Application EP-A-0239969, which describes a form of electrode covered by a membrane comprising an electroconductive film prepared by electrolytic oxidation of an electropolymerisable monomer and having at least one functional group capable of binding an antigen or antibody to the film. This construction is stated to be useful as an immunosensor.
(b) European Patent Application EP-A-0186210, which describes an ion sensor comprising a reversible redox polymer film formed directly on a conductor base. The redox film may be made by electrolytic oxidation of a variety of materials of hydroxy and/or amino-aromatic types. It is stated that this film is dense and does not permit permeation of oxygen molecules even if the film is thin, so the functioning depends upon the incorporation of an ion-carrier substance. The ion-carrier substance has to be intimately incorporated and can be chosen to be selective to ions of choice.

One use for such electrodes is in the analysis of blood or serum, to detect amounts of components which may be very small but also very significant for diagnostic purposes. Especially, it is often important to measure hydrogen peroxide -- which may be generated in situ as a result of the action of cellular enzymes (as present in leukocytes) or an immobilised or soluble oxidase enzyme. Oxidases are used as reagents in various biological media, and so it is often desirable to be able to detect their activity.

Unfortunately, biological fluids, especially blood and serum, contain a variety of components which interfere with these methods, and especially the electrolytic ones. These result in interference or electrode fouling, so that the measurements become inaccurate or even impossible to make. The presence of a variety of drugs, which the patient may have used, also can interfere seriously with the accuracy of the measurement. Thus, the electrodes suffer from the disadvantages of being subject to interference from a variety of chemical species other than the ones sought, so that the accuracy of the measurements often falls short of the reliability required to be clinically useful.

It has been proposed to reduce this interference in use by lowering the polarising voltage applied to the electrode, but this is not always satisfactory because reduction of the voltage sufficiently to reduce the interference usually makes it impossible to maintain a voltage high enough to achieve the electrode reaction desired for the measurement to be made.

It has also been proposed to reduce this interference by interposing membranes between the electrode and the liquid medium (e.g. serum) being analysed, to exclude interfering components. In this, the membrane acts in a selectively permeable manner, physically holding back some components and allowing others to pass through and reach the electrode. This has not been entirely satisfactory because of the difficulty of finding a membrane material which will have the desired selectivity and also the problems of relying entirely on a fragile barrier.

We have now found that the problem may be overcome by forming a coating on the surface of the electrode itself to act as a selectively permeable barrier for undesirable components of the liquid medium being analysed. Such a coating can be made by electrochemical oxidation of certain phenolic materials in situ at the electrode surface.

Thus according to our invention we provide an improved method for making an electrode having a coating of selective permeability, which comprises electrolytically oxidising a phenolic compound at the surface of the said electrode, whereby selectivity can be varied in a controlled manner.

According to our invention, we also provide new electrodes having a coating of an oxidised phenolic compound on their surface which coating acts as a selectively permeable barrier.

The deposit on the electrode surface, though formed by electrolytic oxidation, may sometimes be referred to as a "polymerised phenol" though this term may be not entirely appropriate. It is referred to herein as an "oxidised phenol."

The electrode itself may be any electrode having the properties of an anode at which the current for peroxide or any other species which may be sought may be determined. It is preferably platinum metal, but it may be any other conventional forms for example gold or glassy carbon.

The phenolic compound may be substituted (for example with alkyl groups, halogens, amino groups, carboxylic acid groups, or combinations of these) but should allow ortho- to para- radical polymerisation following generation of a radical phenol electrochemically, and preferably any substituents should be ones not causing major steric hindrance. The term phenolic compound or "phenol" is also used here to include polyhydric phenolic compounds (for example catechol) and polycyclic phenolic compounds (for example naphthols), which may if desired be substituted, as mentioned above. Especially useful examples of phenolic compounds are phenol itself and dopamine.

The anode may be prepared for the coating operation by thorough cleaning, by any procedure which will remove all adherent material from its surface and leave the surface (usually a metal) clean and ready for re-use. Suitable cleaning media for platinum and gold include fine abrasives, for example alumina, and chemical reagents, for example nitric acid.

The coated electrode can usually be used for over 1 to 2 days, or even longer, before it becomes less efficient and needs to be treated to restore its efficiency. When this is required, as indicated by an enhanced current signal to hydrogen peroxide or to interferents, the re-coating can be readily carried out by direct repetition of the initial coating procedure or by first ensuring that the electrode surface is cleaned thoroughly. The cleaned surface can then be re-coated, by repetition of the procedure described herein.

The formation of the coating on the electrode surface may be achieved by making the electrode the anode in an electrical circuit and immersing the electrode in a liquid medium containing the said phenolic compound, and then applying a voltage to the electrode sufficient to oxidise the phenolic compound at its surface. This procedure can be continued until the thickness of the coating on the electrode surface is sufficient. The resulting film is usually very thin, for example less than 0.1 µm or even less than 0.01 µm.

Conveniently, the voltage applied to form the coating or film is in the range + 0.4 to + 0.8 volts or higher, if so desired (commonly about + 0.6 volt), as measured against a silver/silver chloride electrode, but voltages outside this range may be used if desired. In general, using a lower voltage requires a longer deposition time, and the properties of the deposited film may be modified or controlled by the voltage level used to form it.

The liquid medium containing the phenolic compound may be at any convenient pH, with more efficient polymerisation of the phenolic compound usually being achieved at the more alkaline pH values. A convenient pH, we find, is approximately 7.4. Likewise, the liquid medium may contain a concentration of the phenolic compound which may vary over a considerable range, for example in the range of 1 to 50 mM (though concentrations outside this range may be used if desired). For example, a convenient concentration is approximately 5 mM of phenol (or dopamine) but this is not critical.

The method may be used to produce new electrodes which are useful for the analysis in a biological environment, for example in biological fluids and under biological conditions. Especially, it provides electrodes which are useful for the measurement of the components of blood and serum while eliminating interferents.

By controlled film deposition, it is possible to form an array of cross-reacting sensors which enable the user to "pattern recognise" some analytes.

In use, the electrode may be immersed in a sample of the fluid (e.g. blood) and then linked with a suitable cathode in conventional manner. Measurement of the voltage, current and the like may be taken and the measurements taken and recorded as desired, intermittently or continuously. For this, conventional apparatus may be used.

The method can be carried out using conventional electrodes, for example as in an oxygen cell, but with the anode as the platinum metal and the cathode as the silver/silver chloride electrode (which also acts as a pseudo-reference electrode). A three-electrode cell of working/reference/counter electrodes can also be employed if desired for the coating procedure using the phenolic compound.

Operating in the amperometric mode, the use of the electrode as anode in a liquid medium as electrolyte displays a high degree of selectivity to hydrogen peroxide and is exceptionally free from interference from the presence of a number of compounds which normally are prone to interfere considerably, for example ascorbate, urate, paracetamol and other components in serum. Selectivity is superior to that obtainable using the conventional cellulose acetate membranes, as in oxidase-based bio-sensors and has the added advantage of much faster response times.

To improve the selectivity attainable, and also to protect the coated electrode from possible damage, e.g. attrition or erosion which could break the continuity of the coating, we prefer to surround the electrode with a membrane to avoid mechanical damage to the coating.

This membrane may be composed of any permeable polymer layer, for example a dialysis membrane or a polycarbonate with pores, depending upon the application intended.

According to our invention we also provide a new composite electrode device comprising as anode an electrode having a coating of an oxidised phenolic compound on its surface (as more fully described above) in conjunction with a second electrode as cathode, with the two electrodes being held together in an assembly which prevents direct electrical contact between them.

In one construction, a cathode surrounds the anode, and is spaced from it by a non-conductive spacer element which also secures a membrane in place around the anode and the cathode in a sealed relationship which provides a means for retaining an appropriate electrolyte between the membrane and the anode and also provides some added physical protection of the anode against surface damage.

According to our invention we also provide an improved method for the detection and measurement of hydrogen peroxide, which comprises adding a sample material under examination to a liquid medium and then detecting, by electrolytic analysis (especially amperometrically) the amount of hydrogen peroxide present as such or indirectly formed (e.g. from an enzymatic reaction) using an electrode having a coating of selective permeability made by electrolytically oxidising a phenolic compound at the surface of the said electrode.

This can be used to measure the amount of hydrogen peroxide, etc., however it may be present. Thus it may be present as such or generated in situ, for example by enzymatic action, especially an enzymatic reaction at or near the electrode surface.

When the hydrogen peroxide to be measured is generated by enzymatic action, the enzymes used (usually oxidases) may be any of those known in the art, and they may be located anywhere in the system which is found to be convenient, provided they are detrimental to the function of the "oxidised phenol" coating. Thus the enzyme may be free (e.g. in solution) or immobilised, and may even be made part of an electrode or sensor assembly. Such enzymes, methods for immobilising them, and forms of construction for using them with or as part of an electrode or sensor system are well known in the art.

The sample may especially be a biological one, for example a biological fluid and especially blood or serum.

The liquid medium, which acts as the electrolyte in the cell containing an electrode of the present invention, may be any in which the desired action of the enzyme can take place and in which the components are soluble. Thus, it need not necessarily be aqueous, and organic solvents (as such or as mixtures with each other and/or water) may be used provided that they do not interfere with the desired electrochemical processes and the deposition of the film on the anode. Preferably, the electrolyte is an aqueous buffer solution (most conveniently a phosphate buffer solution) which maintains the pH of the solution and added sample at a desired level. This pH is not critical in the broad sense, as it depend principally on the pH needs of the enzyme for its activity. An example is thus approximately pH 7.4 (the pH of blood).

The proportion of the hydrogen peroxide or peroxide precursor in it is preferably in the range 10⁻⁵ to 10⁻² M of the liquid medium, though larger or smaller proportions may be used if desired. Water-miscible organic solvents may be present if they do not interfere with the essential oxidation or reduction processes involved.

The procedure and apparatus to be used for carrying out the amperometric electrolytic analysis, and for displaying and/or recording the resulting measurements, may be any of those which are known or conventional for the purpose.

According to our invention we also provide an improved electrode system for use in amperometric analysis which comprises an electrode as defined above, i.e. an electrode having a coating of selective permeability comprising a film of an oxidised phenolic compound on its surface.

The sample of the blood or serum for examination may be obtained by standard methods. The quantity of the blood or serum added to the liquid medium should be such as to provide a concentration in the range 10⁻⁵ to 10⁻² M/l in the liquid medium.

The procedure can be much improved by surrounding the anode with a membrane which serves to reduce the access of undesired materials to the anode surface. This membrane may act by virtue of its effectiveness as a dialysis membrane (i.e. by impeding passage of larger molecules) or as a perm-selective membrane (which controls the passage of molecules or ions according to their properties other than just physical size). These function to reduce the fouling of the electrode and increase accuracy. Such a membrane may be for example composed of conventional materials, especially a polycarbonate, cellulose acetate, nitrocellulose, or the like.

A convenient example is an outer polycarbonate membrane of thickness 0.03 to 0.05 µm, which allows the phenolic compound to pass through to the platinum surface. This (a) prevents any damage or scratching of the surface films and (b) allows slower, more controlled deposition of the phenolic compounds.

The physical state of the membrane may be controlled in terms of such features as thickness, pore size, and any other feature which may have an effect on its permeability so as to control diffusion to the electrode surface to prevent excess of solute exposure.

In use, the polarised electrodes (or a composite structure comprising both anode and cathode in a single assembly) may be immersed in a predetermined volume of the buffer solution to which can be added the sample (for example a sample of blood or serum) under test, so that the amperometric measurements made and compared before and after the addition of the sample under test. The procedure may also be calibrated by use of solutions containing known amounts of any components sought or believed to interfere, so that the results can be properly standardised and the apparatus calibrated.

Conventional apparatus may be used, for the cell, electrodes and the measurement and recording of the current-time relationships for the samples under test. Measurements may be made continuously or intermittently, as desired.

The membrane and/or anode may be prepared for use in the analytical process of the invention by soaking it, when it is in place around the anode, in a solution corresponding to the electrolyte medium before the blood/serum sample is added.

The advantages of the invention are that the interference from other components of blood and serum can be eliminated or at least greatly reduced to a level at which they no longer interfere with the measurement to a degree which renders the measurements unreliable or misleading under clinical conditions.

## Claims

1. Method for the detection and measurement of hydrogen peroxide which comprises examining a sample of material in a liquid medium and detecting by electrolytic analysis the amount of hydrogen peroxide present, characterised by using an electrode having a coating of selective permeability made by electrolytically oxidising a phenolic compound at the surface of the said electrode.

2. Method as claimed in Claim 1 wherein the coating on the electrode surface is formed by making the electrode the anode in an electrical circuit and immersing the electrode in a liquid medium containing the phenolic compound, and then applying a voltage to the electrode sufficient to oxidise the phenolic compound at its surface,

3. Method as claimed in Claim 1 or Claim 2 wherein the thickness of the coating on the electrode surface is 0.1 µm or less.

4. Method as claimed in Claim 1 or Claim 2 wherein the phenolic compound is phenol itself or dopamine.

5. Method as claimed in any of Claims 1 to 3 wherein the electrode carrying the coating is made of platinum.

6. Method as claimed in any of Claims 1 to 4 wherein the voltage applied to form the surface coating is in the range +0.4 to +0.8 volts against a silver/silver chloride electrode.

7. Method as claimed in any of Claims 1 to 5 wherein the electrode is surrounded with a permeable membrane.

8. Method as claimed in any of Claims 1 to 6 wherein the sample material under examination is a biological sample, for example a biological fluid.

9. Method as claimed in Claims 1 to 8 wherein the hydrogen peroxide is formed in situ from another compound.

10. Method as claimed in Claim 9 wherein the hydrogen peroxide is formed indirectly by enzymatic action from another compound.

11. An electrode having a surface coating which is made by electrolytically oxidising a phenolic compound at the surface of the said electrode, said coating acting as a selectively permeable barrier.

12. An electrode as claimed in Claim 11 wherein the surface coating has a thickness less than 0.1 µm.

13. An electrode assembly comprising as anode an electrode as claimed in Claim 11 or Claim 12 in conjunction with a second electrode as cathode, the two electrodes being held together in an assembly which prevents direct electrical contact between them.

14. An electrode or electrode assembly as claimed in Claims 11 to 13 wherein the anode is accompanied by a membrane which is a dialysis or perm-selective membrane which serves to reduce the access of undesired materials to the anode surface.

15. An electrode or electrode assembly as claimed in Claim 14 wherein the dialysis or perm-selective membrane has a thickness of 0.03 to 0.05 µm.

16. An electrode assembly as claimed in Claims 11 to 15 wherein the cathode surrounds the anode, and is spaced from it by a non-conductive spacer element which also secures a membrane in place around the anode in a sealed relationship which provides a means for retaining an appropriate electrolyte between the membrane and the anode and also provides some added physical protection of the anode against surface damage.

17. An electrode assembly as claimed in Claim 16 wherein the electrolyte is an aqueous buffer solution to maintain a desired pH.

## Patentansprüche

1. Verfahren zum Nachweis und zur Bestimmung von Wasserstoffperoxid, bestehend aus der Untersuchung einer Stoffprobe in einem flüssigen Medium und Nachweis der vorhandenen Menge Wasserstoffperoxid durch elektrolytische Analyse, dadurch gekennzeichnet, daß eine Elektrode mit einem selektiv durchlässigen Überzug, der durch elektrolytisches Oxidieren einer phenolischen Verbindung an die Oberfläche der genannten Elektrode hergestellt wird, verwendet wird.

2. Verfahren nach Anspruch 1, wobei der Überzug auf der Elektrodenoberfläche dadurch gebildet wird, daß die Elektrode in einem elektrischen Stromkreis zur Anode gemacht wird, und die Elektrode in ein Flüssigkeitsmedium eingetaucht wird, das die phenolische Verbindung enthält, und dann eine Spannung auf die Elektrode gelegt wird, die ausreicht, um die phenolische Verbindung auf ihrer Oberfläche zu oxidieren.

3. Verfahren nach Anspruch 1 oder 2, wobei die Beschichtungsdicke auf der Elektrodenfläche 0,1 µm oder weniger beträgt.

4. Verfahren nach Anspruch 1 oder 2, wobei die phenolische Verbindung Phenol selbst oder Dopamin ist.

5. Verfahren nach Anspruch 1 bis 3, wobei die Elektrode, die die Beschichtung trägt, aus Platin hergestellt ist.

6. Verfahren nach Anspruch 1 bis 4, wobei die zur Bildung der Oberflächenbeschichtung angelegte Spannung sich im Bereich + 0,4 bis + 0,8 Volt gegen eine Silber/Silberchloridelektrode befindet.

7. Verfahren nach Anspruch 1 bis 5, wobei die Elektrode von einer durchlässigen Membrane umgeben ist.

8. Verfahren nach Anspruch 1 bis 6, wobei das zu untersuchende Probematerial eine biologische Probe ist, beispielsweise einer biologischen Flüssigkeit.

9. Verfahren nach Anspruch 1 bis 8, wobei das Wasserstoffperoxid in situ von einer anderen Verbindung gebildet ist.

10. Verfahren nach Anspruch 9, wobei das Wasserstoffperoxid indirekt durch Enzymwirkung von einer anderen Verbindung gebildet wird.

11. Elektrode mit einer Oberflächenbeschichtung, die durch elektrolytisches Oxidieren einer phenolischen Verbindung auf die Oberfläche der genannten Elektrode hergestellt wird, wobei die genannte Beschichtung als eine selektiv durchlässige Sperrschicht wirkt.

12. Elektrode nach Anspruch 11, wobei die Oberflächenbeschichtung eine Dicke von weniger als 0,1 µm hat.

13. Elektrodenzusammenbau bestehend aus einer Elektrode als Anode, wie in Anspruch 11 oder Anspruch 12 beansprucht in Verbindung mit einer zweiten Elektrode als Kathode, wobei die zwei Elektroden in einem Zusammenbau zusammengehalten werden, was direkten elektrischen Kontakt zwischen ihnen verhindert.

14. Eine Elektrode oder ein Elektrodenzusammenbau nach Anspruch 11 bis 13, wobei die Anode von einer Membrane begleitet ist, die eine Dialyse- oder Permselektiv-Membrane ist, die dazu dient, den Zugang unerwünschter Materialien zur Anodenoberfläche zu reduzieren.

15. Eine Elektrode oder ein Elektrodenzusammenbau nach Anspruch 14, wobei die Dialyse- oder Permselektiv-Membrane eine Dicke von 0,03 bis 0,05 µm hat.

16. Ein Elektrodenzusammenbau nach Anspruch 11 bis 15, wobei die Kathode die Anode umgibt, und ihr Abstand durch ein nichtleitendes Abstandselement gehalten wird, das auch eine Membrane um die Anode in einem abgedichteten Verhältnis an Ort und Stelle festhält, was ein Mittel zum Zurückhalten eines passenden Elektrolyten zwischen der Membrane und der Anode bietet und außerdem der Anode gewissen zusätzlichen physikalischen Schutz gegen Oberflächenbeschädigung liefert.

17. Ein Elektrodenzusammenbau nach Anspruch 16, wobei der Elektrolyt eine wäßrige Pufferlösung ist, um einen erwünschten pH-Wert aufrechtzuerhalten.

## Revendications

1. Procédé pour détecter et mesurer du péroxyde d'hydrogène, comprenant l'examen d'un échantillon de matériau en milieu liquide, et la détermination par analyse électrolytique de la quantité de péroxyde d'hydrogène présent, caractérisé en ce qu'on utilise une électrode qui présente un revêtement de perméabilité sélective, obtenu en oxydant par voie électrolytique un composé phénolique à la surface de ladite électrode.

2. Procédé selon la revendication 1, dans lequel le revêtement sur la surface de l'électrode est formé en faisant de l'électrode l'anode d'un circuit électrique et en immergeant l'électrode dans un milieu liquide contenant le composé phénolique, puis en appliquant à l'électrode une tension suffisante pour oxyder le composé phénolique à sa surface.

3. Procédé selon la revendication 1 ou 2,dans lequel l'épaisseur du revêtement à la surface de l'électrode est de 0,1 µm ou moins.

4. Procédé selon la revendication 1 ou 2, dans lequel le composé phénolique est le phénol lui-même ou la dompamine.

5. Procédé selon l'une quelconque des revendications 1 à 3, dans lequel l'électrode portant le revêtement est en platine.

6. Procédé selon l'une quelconque des revendications 1 à 4, dans lequel la tension appliquée pour former le revêtement de surface est comprise entre +0,4 et +0,8 volts par rapport à une électrode argent/chlorure d'argent.

7. Procédé selon l'une quelconque des revendications 1 à 5, dans lequel l'électrode est entourée d'une membrane perméable.

8. Procédé selon l'une quelconque des revendications 1 à 6, dans lequel l'échantillon de matériau à examiner est un échantillon biologique, par exemple un fluide biologique.

9. Procédé selon les revendications 1 à 8, dans lequel le péroxyde d'hydrogène est formé "in situ" à partir d'un autre composé.

10. Procédé selon la revendication 9, dans lequel le péroxyde d'hydrogène est formé indirectement par action enzymatique, à partir d'un autre composé.

11. Electrode ayant à sa surface un revêtement obtenu en oxydant par voie électrolytique un composé phénolique à la surface de ladite électrode, ledit revêtement agissant comme une barrière de perméabilité sélective.

12. Electrode selon la revendication 11, dans laquelle le revêtement de surface a une épaisseur inférieure à 0,1 µm.

13. Assemblage d'électodes comprenant comme anode une électrode telle que définie à la revendication 11 ou à la revendication 12, conjointement avec une seconde électrode comme cathode, les deux électrodes étant maintenues ensemble dans un assemblage qui empêche le contact électrique direct entre elles.

14. Electrode ou assemblage d'électrodes selon les revendications 11 à 13, dans laquelle ou lequel l'anode est accompagnée d'une membrane qui est une membrane de dialyse ou à perméabilité sélective, servant à réduire l'accès de matières non désirées à la surface de l'anode.

15. Electrode ou assemblage d'électrodes selon la revendication 14, dans laquelle ou lequel la membrane de dialyse ou à perméabilité sélective présente une épaisseur de 0,03 à 0,05 µm.

16. Assemblage d'électrodes selon les revendications 11 à 15, dans lequel la cathode entoure l'anode et est espacée de celle-ci par un élément de séparation non-conducteur, lequel maintient également en place une membrane autour de l'anode, en position relative fixe, ce qui crée un moyen pour retenir un électrolyte approprié entre la membrane et l'anode et qui assure aussi une certaine protection physique supplémentaire de l'anode contre des dommages superficiels.

17. Assemblage d'électrodes selon la revendication 16, dans lequel l'électrolyte est une solution aqueuse tampon, pour maintenir un pH désiré.
